# EUROPEAN PATENT SPECIFICATION

(11) **EP 3 236 950 B1**
(45) Date of publication and mention of the grant of the patent: **23.11.2022**
(21) Application number: 15817391.4
(22) Date of filing: 24.12.2015
(51) Int. Cl.: A61K 9/20, A61K 31/4422, A61K 31/4184

(54) **PHARMACEUTICAL COMPOSITION COMPRISING CANDESARTAN OR PHARMACEUTICALLY ACCEPTABLE SALTS OR ESTERS THEREOF AND AMLODIPINE OR PHARMACEUTICALLY ACCEPTABLE SALTS THEREOF**
PHARMAZEUTISCHE ZUSAMMENSETZUNG MIT CANDESARTAN ODER PHARMAZEUTISCH AKZEPTABLEN SALZEN ODER ESTERN DAVON UND AMLODIPIN ODER PHARMAZEUTISCH AKZEPTABLEN SALZEN DAVON
COMPOSITION PHARMACEUTIQUE COMPRENANT DU CANDÉSARTAN OU DES SELS OU ESTERS PHARMACEUTIQUEMENT ACCEPTABLES DE CE DERNIER, ET DE L'AMLODIPINE OU DES SELS PHARMACEUTIQUEMENT ACCEPTABLES DE CE DERNIER

(30) Priority: 24.12.2014 SI 201400455
(43) Date of publication of application: 01.11.2017
(73) Proprietor: KRKA, d.d., Novo mesto, 8000 Novo mesto (SI)
(72) Inventor: GERMAN, Tamara, 8000 Novo mesto (SI); KAPELE, Tomaz, 8000 Novo mesto (SI); KORASA, Klemen, 8000 Novo mesto (SI); UREK-BLATNIK, Sandra, 8000 Novo mesto (SI); VRECER, Franc, 8000 Novo mesto (SI)
(74) Representative: Hoffmann Eitle
(86) International application number: PCT/EP2015/081234
(87) International publication number: WO 2016/102705

(56) References cited:
- EP-A1- 2 106 789
- WO-A1-2008/044862
- WO-A1-2014/097209

## Description

The invention relates to a solid pharmaceutical composition comprising at least one portion comprising candesartan cilexetil and at least one another portion comprising amlodipine besylate in a fixed dosage form.

### Background of the invention

Candesartan with chemical name 1-(cyclohexyloxycarbonyloxy)ethyl 1-((2'-cyanobiphenyl-4-yl)methyl)-2-ethoxy-1H-benzo(d)imidazole-7-carboxylate is a selective antagonist of subtype 1 of the angiotensin II receptor that was originally disclosed in EP 0 459 136 and EP 0 720 982. It falls in the class of drugs of benzimidazole-7-carboxylic acids and salts or esters thereof. These agents exhibit a strong and more effective hypotensive action and are less likely to cause coughing as a side effect when compared to the class of ACE inhibitors. Candesartan cilexetil (a prodrug of candesartan) is used for the treatment of essential hypertension and for the treatment of patients with heart failure and impaired left ventricular systolic function.

In general, the preparation of stable formulation of candesartan or pharmaceutically acceptable salts or esters thereof for effective oral administration is complicated by the unique physical and chemical properties of the compound. It is known that candesartan cilexetil is stable against temperature, moisture and light when it is stored individually in the solid state but when it is incorporated into a pharmaceutical composition by means of compression the decomposition of the active ingredient is observed. Up to now this problem was solved by several different ways with the main stress on solving the stability problems. A number of different types of materials and approaches has already been tried in order to find the solution for stabilizing candesartan cilexetil in a solid pharmaceutical composition. Another development challenge is the low bioavailability/solubility of candesartan or pharmaceutically acceptable salts or esters thereof. From clinical data it has been estimated that only between 15 and 40 % of the candesartan or pharmaceutically acceptable salts or esters thereof contained in conventional solid dosage forms is becoming physiologically available and active.

EP 0 546 358 A1 solves the problem of decomposition of candesartan and/or its pharmaceutically acceptable salts or esters by incorporating an oily substance having a low melting point into the formulation. EP 1 952 806 A1 relates to a process for the preparation of adsorbates of candesartan cilexetil and/or of its hydrates, wherein one starts from a solution consisting of candesartan cilexetil and/or its hydrates in at least one organic solvent, disperses in it an adsorbing material and removes the solvent and wherein the adsorbing materials are selected in particular from the group of carbohydrates and related polyols, including celluloses, cellulose derivatives, sugars, sugar derivatives, cyclodextrins, starches, starch derivatives, polydextroses, or mixtures thereof. WO 2006/079496 discloses a solid pharmaceutical composition comprising candesartan cilexetil characterized in that it comprises from 2 to 20 % of a hydrophilic substance with hydrocolloidal properties. WO 2007/147514 relates to a solid stable composition of candesartan cilexetil characterized in that in this composition the surface of candesartan cilexetil is provided with a coating comprising a compound or a mixture of compounds chosen from tri(C1-C6)alkyl citrate, di(C1-C6)alkyl phthalate, di(C1-C6)alkyl sebacate and polydimethyl-siloxanes, wherein this compound or the mixture of these compounds by itself forms the coating or represents a substantial constituent of the coating. EP 2 165 702 A1 discloses the composition comprising candesartan cilexetil and its pharmaceutically acceptable salts with at least one surfactant selected from the group of anionic surfactants, preferably sodium docusate and optionally at least a second surfactant, preferably sodium lauryl sulfate.

Amlodipine exerts antihypertensive action by reducing the availability of calcium ions for muscular contraction and, therefore, resulting in decreased peripheral vascular resistance and reduced blood pressure. Amlodipine, the chemical name of which is 3-ethyl 5-methyl 2- [ (2-aminoethoxy)methyl]-4-(2-chlorophenyl)-6-methyl-1,4-dihydropyridine-3, 5-dicarboxylate is a long-acting calcium channel blocker (dihydropyridine class) and originally disclosed in EP 0 089 167 is used as an antihypertensive and in the treatment of angina. Amlodipine is commercially available in the form of maleate, besylate or mesylate salt. It is known that amlodipine or pharmaceutically acceptable salts thereof is susceptible to hydrolysis when exposed to an alkaline medium. In addition, amlodipine is an amine compound which is incompatible with some excipients such as lactose or polyethylene glycol.

Guidelines for the Management of Hypertension (Journal of Hypertension 1999, 17: 151-183) discloses that a combination of drugs from different drug classes is used for an improved efficacy in the treatment of high blood pressure and that effective drug combinations utilize drugs from different classes in order to obtain the additive hypotensive effect that comes from combining drugs with different primary actions.

Therefore, as a drug therapy exerting strong hypotensive effect, a combination composition comprising candesartan or pharmaceutically acceptable salts or esters thereof and at least one calcium channel blocker can be used. Certain pharmaceutical combination compositions have already been described in the prior art.

WO 2010/126168 discloses the composition comprising candesartan cilexetil and amlodipine besylate with sugar alcohol such as for example D-mannitol and polyethylene glycol prepared by granulation. All examples show good dissolution property of candesartan cilexetil in comparison to comparative example 1 comprising lactose hydrate. However, it is known that mannitol can affect the intestine motility and besides it may cause mild laxative effect, thus mannitol is not optimal diluent option for application in the pharmaceutical dosage forms.

CN 103705513 A1 describes a pharmaceutical composition comprises candesartan cilexetil, amlodipine besylate, mannitol and polyethylene glycol and high-substituted hydroxypropyl cellulose prepared by wet granulation and tableting.

WO 2014/097209 relates to a combination pharmaceutical composition comprising candesartan cilexetil and amlodipine or a pharmaceutically acceptable salts thereof comprising granulate with candesartan cilexetil and a mixture of hydroxypropyl celluloses as a binder wherein at least one is in the form of an aqueous solution and an extragranular phase comprising a powder blend with amlodipine or a pharmaceutically acceptable salts and excipients. The mixture of different hydroxypropyl celluloses ensures desired bioavailability and stability of the composition.

Phamaceutical compositions comprising candesartan cilexitil and amlodipine or pharmaceutically acceptable salts thereof are disclosed also in EP 2 106 789 and WO 2014/097209.

In the pharmaceutical compositions of the prior art, complex fixed dose formulations of candesartan cilexetil and amlodipine or a pharmaceutically acceptable salts thereof are necessary to address the incompatibility of two active agents and to stabilize the compositions. These complex compositions require the use of substantial amounts of excipients and the preparation of such compositions involves complex and costly processing steps.

It is therefore an object of the present invention to provide a solid pharmaceutical composition comprising candesartan cilexitil and amlodipine besylate in a fixed dosage form avoiding the above-discussed drawbacks and which does not only exhibit high chemical and physical stability as well as an appropriate dissolution profile and bioavailability but can be manufactured using a simple and economical process. The pharmaceutical composition according to the present invention shows excellent dissolution rate and stability, the preparation method is simple and convenient, production cost is low and the pharmaceutical composition is suitable for large-scaled industrialized production.

This objective is surprisingly achieved by the pharmaceutical composition comprising candesartan cilexitil and amlodipine besylate present in different portions of the pharmaceutical composition as specified in the appended claims. More particularly, active substances are separated by means of layers or units in the form of tablets. Even more particularly, the pharmaceutical composition according to the present invention comprising candesartan cilexetil and amlodipine besylate is formulated in the form of a tablet comprising two layers wherein active substances are present in a different layer.

The present invention also relates to a process for the preparation of said pharmaceutical composition comprising candesartan cilexitil and amlodipine besylate in a fixed dosage form as specified in the appended claims.

### Summary of the invention

The present invention relates to a pharmaceutical composition comprising candesartan cilexitil and amlodipine besylate as specified in the appended claims.

The present invention relates to a process for the preparation of a pharmaceutical composition according to the present invention as specified in the appended claims.

### Detailed description of the invention

The present invention relates to a pharmaceutical composition comprising candesartan cilexetil and amlodipine besylate having a composition selected from the embodiments a to f as specified in the table below,

| Embodiment | a [mg] | b [mg] | c [mg] | d [mg] | e [mg] | f [mg] |
|---|---|---|---|---|---|---|
| Candesartan Cilexetil | 16.0 | 16.0 | 16.0 | 16.0 | 16.0 | 16.0 |
| Lactose Monohydrate | 177.8 | / | 177.8 | 177.8 | 177.8 | 81.0 |
| Cellulose, Microcrystalline | / | 177.8 | / | / | / | / |
| Maize Starch | 40.0 | 40.0 | 40.0 | 40.0 | 40.0 | 20.0 |
| Ferric oxide yellow | 0.06 | 0.06 | 0.06 | 0.06 | 0.06 | 0.06 |
| Ferric oxide red | 0.12 | 0.12 | 0.12 | 0.12 | 0.12 | 0.12 |
| Macrogol 8000 | 5.2 | 5.2 | 5.2 | 5.2 | 5.2 | 2.6 |
| Hydroxypropyl cellulose (Klucel^{™} EF) | | | 16.0 | 16.0 | 16.0 | 8.0 |
| Hydroxypropyl cellulose (Klucel^{™} LF) | 16.0 | 16.0 | | | | |
| Water, Purified* | q.s. | q.s. | q.s. | q.s. | q.s. | q.s. |
| Calcium carboxymethyl cellulose | 4.0 | 4.0 | / | 4.0 | 4.0 | 2.0 |
| Sodium carboxymethyl cellulose | / | / | 4.0 | / | / | / |
| Magnesium stearate | 0.8 | 0.8 | 0.8 | 0.8 | 0.8 | 0.4 |
| Amlodipine besylate | 13.9 | 13.9 | 13.9 | 13.9 | 13.9 | 13.9 |
| Cellulose, Microcrystalline | 180.9 | 180.9 | 180.9 | 180.9 | 180.9 | 59.1 |
| Starch, Pregelatinised | / | 60.0 | 60.0 | 60.0 | 60.0 | 18.0 |
| Maize Starch | 60.0 | / | / | / | / | / |
| Sodium Starch Glycolate | 21.00 | 21.00 | 21.00 | 21.00 | / | 7.5 |
| Potassium polacrilin | / | / | / | / | 21.00 | / |
| Silica, Colloidal Anhydrous | 1.25 | 1.25 | 1.25 | 1.25 | 1.25 | 0.50 |
| Magnesium Stearate | 3.0 | 3.0 | 3.0 | 3.0 | 3.0 | 1.0 |

| | | | | | | |
|---|---|---|---|---|---|---|
| *Not present in the final dosage form. Evaporates during the process. | | | | | | |

which is obtainable by the following manufacturing method:
(i) homogeneously mixing amlodipine besylate, microcrystalline cellulose, binder (maize starch (embodiment a) or pregelatinised starch (embodiments b-f), sodium starch glycolate (embodiments a-d and f) or sodium carboxymethyl cellulose (embodiment e)), silica, colloidal anhydrous and magnesium stearate to obtain the powder blend;
(ii) separately dissolving hydroxypropyl cellulose in water, then adding Macrogol 8000 and dispersing candesartan cilexetil in said solution to obtain a water dispersion;
(iii) granulating lactose monohydrate (embodiments a and c-f) or microcrystalline cellulose (embodiment b), ferric oxide, and maize starch with said water dispersion in a fluid bed granulator;
(iv) adding calcium carboxymethyl cellulose (example 2-3 and 5-7) or sodium carboxymethyl cellulose (example 4) and magnesium stearate to the granules of step (iii) to obtain a granulated blend; and
(v) compressing both blends of steps (i) and (iv) into bilayer tablets.

In the pharmaceutical composition according to the present invention both active ingredients candesartan cilexitil and amlodipine besylate remain stable during the manufacturing process and during storage. In addition, both active ingredients exhibit the same dissolution profile and bioavailability in comparison to the pharmaceutical composition comprising each single active ingredient.

Moreover, the pharmaceutical composition according to the present invention avoids the influence of variability of particulate properties of the specific single type binder, such as for example parameters of particle size distribution, such as for example d₁₀, d₅₀, or d₉₀, average particle size, particle morphology and similar. With said better pharmaceutical and biopharmaceutical properties and quality of the final pharmaceutical composition such as stability and/or pharmacokinetic properties of active ingredient(s) can be achieved.

Candesartan cilexetil used in the present invention can be in an amorphous or crystalline form. As used herein, the term "amorphous" includes amorphous and partly crystallized forms. Amorphous candesartan cilexetil can be obtained by methods generally known in the art such as but not limited to freeze drying of aqueous solutions, preparing a solid dispersion by coating of carrier particles with drug containing coating dispersion in a fluidized bed, preparing the solid dispersion by hot melt processes such as hot melt extrusion and/or termoplastic granulation, spray drying of drug and carrier solution in appropriate solvent, preparing drug complexes with carriers such cyclodextrins and solvent deposition on sugar pellets or other carriers. Candesartan cilexetil can be prepared by any method known from the state of the art as for example disclosed in EP0459136A1, EP0720982A1, WO95/32962, EP0881212A1, EP1191998A1, CN1361101A1, WO2003/014112, CN1510031A1, WO2005/021535, WO2005/037821, WO2005/051928, EP1555260A1, WO2005/077941, WO2005/111021, WO2006/015134, WO2006/050922, WO2006/063578, WO2006/067215, WO2006/076710, WO2006/122254, WO2007/074399, WO2007/014412, WO2007/042161, WO2007/094015, WO2008/012371, WO2008/012372, WO2008/062047, WO2008/129077, WO2009/007986, WO2011/080684, WO2011/145100, WO2013/041944 etc.

Moreover, candesartan cilexetil can be in any known stable polymorphic or hydrate form known from the state of the art as for example disclosed in EP 0 459 136 A1, EP 0 720 982 A1, WO2004085426, WO 2005077941, WO2005123721, WO2008035360, WO2011092666.

The amount of candesartan cilexitil used in the pharmaceutical composition according to the present invention is as specified in the appended claims.

Amlodipine is in the form of besylate salt. Amlodipine or pharmaceutically acceptable salts thereof can be prepared by any method known from the state of the art. Amlodipine besylate can be used either in crystalline or amorphous form.

The amount of amlodipine besylate used in the pharmaceutical composition according to the present invention is as specified in the appended claims.

It was surprisingly found out that the pharmaceutical composition of the present invention exhibits dissolution properties and in vivo performance comparable with individual single drug reference compositions of candesartan cilexetil and amlodipine besylate. In the present invention active ingredients are present in different portions of the pharmaceutical composition which means that candesartan cilexetil is present in one portion and amlodipine besylate is present in another portion. It is known form the state of the art, that physicochemical and biopharmaceutical properties of ingredients of one portion, for example of an individual layer or unit of the pharmaceutical composition per se can still influence the pharmaceutical characteristics of ingredients present in another portion. However, intense research and development activities have led to the present invention of a multilayer or multiunit pharmaceutical combination composition with pharmaceutical properties comparable to the individual single drug compositions. The multilayer pharmaceutical composition of the present invention is a bilayer tablet comprising candesartan cilexetil and amlodipine besylate present in separate layers as specified in the appended claims. The comparability of bilayer composition with single dosage compositions of candesartan cilexetil and of amlodipine besylate compositions was proven with in vitro and in vivo tests.

The term "portion" according to the present invention means that active ingredients are not as a whole in intimate contact and that they are substantially separated from each other, for example by means of layers or units in the form of tablets. Thus, as used herein, the term "portion" refers to a physical part of the solid pharmaceutical composition wherein the components of said part are not intimately mixed with the components of another portion or part of the composition. In particular, the interaction between the components of one portion and the components of another portion is reduced, for example by physically separating one portion from another portion by the provision of providing two or more tablet layers into multilayer tablets or two or more units in the multiunit tablets. Thus, by providing the two pharmaceutically active agents in the form of separate portions, contact between the two active agents can be minimized. At best, such contact will be possible at the interface between adjacent portions, whereas contact inside the portions is prevented. The size and shape of the portions is not particularly limited. The portions are present in the form of two separate layers of a tablet.

The pharmaceutical composition of the invention is in the form of a bilayer tablet.

The term "powder blend" according to the present invention means a physical mixture of an active ingredient and one or more pharmaceutically acceptable excipients, which is prepared by blending of an active ingredient and one or more pharmaceutically acceptable excipients with the state of the art blending equipment such as for example container blender, V-shaped blender, biconical blender, conical blender, horizontal blender, and high turbulence blender. The active ingredient is amlodipine besylate.

According to the present invention single type hydroxypropyl cellulose is used in the portion containing candesartan cilexitil.

For example, solutions of hydroxypropyl cellulose with average molecular weight in the range from 50,000 to 1,500,000, preferably from 70.000 to 1,200,000 can be used. Such average molecular weight indications may alternatively refer to the weight average molecular weight or the number average molecular weight of the specified material.

The amount of a specific single type hydroxypropyl cellulose present in the pharmaceutical composition of the present invention is independent on the amount of active ingredient candesartan cilexitil. The amount of hydroxypropyl cellulose is as specified in the appended claims.

The term 'tablet core' according to the present invention means pharmaceutical unit dosage form obtained by compressing granulated blend portion and powder blend or granulate portion by using state of the art tableting equipment. Consequently, the term 'tablet core' does not include any coatings of the tablet.

In the pharmaceutical composition of the present invention the single type hydroxypropyl cellulose is present in the portion comprising the granulated blend comprising candesartan cilexitil.

The pharmaceutical composition according to the present invention further comprises pharmaceutically acceptable excipients as specified in the appended claims.

Another object of the present invention provides a process of the preparation of the pharmaceutical composition as specified in the appended claims.

Wet granulation of candesartan cilexitil is performed by fluid bed technology.

Powder blend comprising amlodipine besylate is prepared by simple blending of individual blend components. Such production approach brings many advantages over indirect manufacturing process, such as granulation process, due to its simplicity, short process times, low energy consumption, little equipment requirements, and low moisture and energy exposure.

### Examples

The present invention can be illustrated in one of its objectives by the following non-limiting examples. All quantities of raw materials in composition for each example are given in amounts for one tablet.

### Comparative example 1

| Comparative Example 1 | |
|---|---|
| Candesartan cilexetil | 16.0 mg |
| Amlodipine besylate | 13.9 mg |
| Lactose | 217.4 mg |
| Maize Starch | 50.8 mg |
| Hydroxypropyl cellulose (Klucel EF) | 10.2 mg |
| Macrogol 8000 | 6.6 mg |
| Purified water | q.s. |
| Calcium carboxymethyl cellulose | 14.2 mg |
| Magnesium stearate | 1.0 mg |

In preparation of Comparative Example 1 hydroxypropyl cellulose was dissolved in water, then Macrogol 8000 was added and candesartan cilexetil was dispersed in said solution to obtain water dispersion. Lactose monohydrate and maize starch were granulated with said water dispersion in a fluid bed granulator. After the granulation step amlodipine besylate, calcium carboxymethyl cellulose and magnesium stearate were added to the granules. The obtained composition was compressed into tablets.

### Examples 2-7

| Example | 2 [mg] | 3 [mg] | 4 [mg] | 5 [mg] | 6 [mg] | 7 [mg] |
|---|---|---|---|---|---|---|
| Candesartan Cilexetil | 16.0 | 16.0 | 16.0 | 16.0 | 16.0 | 16.0 |
| Lactose Monohydrate | 177.8 | / | 177.8 | 177.8 | 177.8 | 81.0 |
| Cellulose, Microcrystalline | / | 177.8 | / | / | / | / |
| Maize Starch | 40.0 | 40.0 | 40.0 | 40.0 | 40.0 | 20.0 |
| Ferric oxide yellow | 0.06 | 0.06 | 0.06 | 0.06 | 0.06 | 0.06 |
| Ferric oxide red | 0.12 | 0.12 | 0.12 | 0.12 | 0.12 | 0.12 |
| Macrogol 8000 | 5.2 | 5.2 | 5.2 | 5.2 | 5.2 | 2.6 |
| Hydroxypropyl cellulose (Klucel^{™} EF) | | | 16.0 | 16.0 | 16.0 | 8.0 |
| Hydroxypropyl cellulose (Klucel^{™} LF) | 16.0 | 16.0 | | | | |
| Water, Purified* | q.s. | q.s. | q.s. | q.s. | q.s. | q.s. |
| Calcium carboxymethyl cellulose | 4.0 | 4.0 | / | 4.0 | 4.0 | 2.0 |
| Sodium carboxymethyl cellulose | / | / | 4.0 | / | / | / |
| Magnesium stearate | 0.8 | 0.8 | 0.8 | 0.8 | 0.8 | 0.4 |
| Amlodipine besylate | 13.9 | 13.9 | 13.9 | 13.9 | 13.9 | 13.9 |
| Cellulose, Microcrystalline | 180.9 | 180.9 | 180.9 | 180.9 | 180.9 | 59.1 |
| Starch, Pregelatinised | / | 60.0 | 60.0 | 60.0 | 60.0 | 18.0 |
| Maize Starch | 60.0 | / | / | / | / | / |
| Sodium Starch Glycolate | 21.00 | 21.00 | 21.00 | 21.00 | / | 7.5 |
| Potassium polacrilin | / | / | / | / | 21.00 | / |
| Silica, Colloidal Anhydrous | 1.25 | 1.25 | 1.25 | 1.25 | 1.25 | 0.50 |
| Magnesium Stearate | 3.0 | 3.0 | 3.0 | 3.0 | 3.0 | 1.0 |

| | | | | | | |
|---|---|---|---|---|---|---|
| *Not present in the final dosage form. Evaporates during the process. | | | | | | |

In preparation of Examples 2-7 amlodipine besylate, microcrystalline cellulose, binder (maize starch (example 2) or pregelatinised starch (example 3-7), sodium starch glycolate (example 2-5 and 7) or sodium carboxymethyl cellulose (example 6)), silica, colloidal anhydrous and magnesium stearate were homogeneously mixed to obtain the powder blend. Separately hydroxypropyl cellulose was dissolved in water, then Macrogol 8000 was added and candesartan cilexetil was dispersed in said solution to obtain water dispersion. Lactose monohydrate (example 2 and 4-7) or microcrystalline cellulose (example 2), ferric oxide, and maize starch were granulated with said water dispersion in a fluid bed granulator. After the granulation step has been performed, calcium carboxymethyl cellulose (example 2-3 and 5-7) or sodium carboxymethyl cellulose (example 4) and magnesium stearate were added to the granules to obtain the granulated blend. Both blends were compressed into bilayer tablets.

### Example 8 (comparative example)

| Example | 8 |
|---|---|
| Candesartan Cilexetil | 16.0 mg |
| Lactose Monohydrate | 81.0 mg |
| Maize Starch | 20.0 mg |
| Ferric oxide red | 0.03 mg |
| Macrogol 8000 | 2.6 mg |
| Hydroxypropyl cellulose (Klucel^{™} EF) | 8.0 mg |
| Water, Purified* | q.s. |
| Calcium carboxymethyl cellulose | 2.0 mg |
| Magnesium Stearate | 0.40 mg |
| Amlodipine besylate | 13.9 mg |
| Cellulose, Microcrystalline | 59.1 mg |
| Starch, Pregelatinised | 18.0 mg |
| Sodium Starch Glycolate | 7.5 mg |
| Silica, Colloidal Anhydrous | 0.5 mg |
| Magnesium Stearate | 1.00 mg |

| | |
|---|---|
| *Not present in the final dosage form. Evaporates during the process. | |

In preparation of Example 8 amlodipine besylate, microcrystalline cellulose, pregelatinised starch, sodium starch glycolate, silica, colloidal anhydrous and magnesium stearate were homogeneously mixed to obtain the powder blend which was compressed into tablets. Separately hydroxypropyl cellulose was dissolved in water, then Macrogol 8000 was added and candesartan cilexetil was dispersed in said solution to obtain water dispersion. Lactose monohydrate, ferric oxide, and maize starch were granulated with said water dispersion in a fluid bed granulator. After the granulation step calcium carboxymethyl cellulose and magnesium stearate were added to the granules to obtain the granulated blend which was compressed into tablet. Tablet comprising powder blend and tablet comprising granulated blend were filled into capsule.

### Example 9 (comparative example)

| Example | 9 |
|---|---|
| Candesartan Cilexetil | 8.0 mg |
| Lactose Monohydrate | 157.9 mg |
| Maize Starch | 40.0 mg |
| Ferric oxide yellow | 0.06 mg |
| Macrogol 8000 | 5.2 mg |
| Hydroxypropyl cellulose (Klucel^{™} EF) | 36.0 mg |
| Water, Purified* | q.s. |
| Calcium carboxymethyl cellulose | 12.0 mg |
| Magnesium Stearate | 0.80 mg |
| Amlodipine besylate | 13.9 mg |
| Cellulose, Microcrystalline | 59.1 mg |
| Starch, Pregelatinised | 18.0 mg |
| Sodium Starch Glycolate | 7.5 mg |
| Silica, Colloidal Anhydrous | 0.5 mg |
| Magnesium Stearate | 1.00 mg |

| | |
|---|---|
| *Not present in the final dosage form. Evaporates during the process. | |

Amlodipine besylate, microcrystalline cellulose, pregelatinised starch, sodium starch glycolate, colloidal anhydrous silica and magnesium stearate were homogeneously mixed to obtain the powder blend. Separately hydroxypropyl cellulose was dissolved in water, then Macrogol 8000 was added and candesartan cilexetil was dispersed in said solution to obtain water dispersion. Lactose monohydrate, ferric oxide, and maize starch were granulated with said water dispersion in a fluid bed granulator. After the granulation step calcium carboxymethyl cellulose and magnesium stearate were added to the granules to obtain the granulated blend. Both blends were compressed into bilayer tablets.

### Example 10 (comparative example)

| Example | 10 |
|---|---|
| Candesartan Cilexetil | 8.0 mg |
| Lactose Monohydrate | 185.9 mg |
| Maize Starch | 40.0 mg |
| Ferric oxide yellow | 0.06 mg |
| Macrogol 8000 | 5.2 mg |
| Hydroxypropyl cellulose (Klucel^{™} EF) | 16.0 mg |
| Water, Purified* | q.s. |
| Calcium carboxymethyl cellulose | 4.0 mg |
| Magnesium Stearate | 0.80 mg |
| Amlodipine besylate | 6.9 mg |
| Cellulose, Microcrystalline | 187.8 mg |
| Starch, Pregelatinised | 60.0 mg |
| Sodium Starch Glycolate | 21.0 mg |
| Silica, Colloidal Anhydrous | 1.3 mg |
| Magnesium Stearate | 3.0 mg |

Amlodipine besylate, microcrystalline cellulose, pregelatinised starch, sodium starch glycolate, colloidal anhydrous silica and magnesium stearate were homogeneously mixed to obtain the powder blend. Separately hydroxypropyl cellulose was dissolved in water, then Macrogol 8000 was added and candesartan cilexetil was dispersed in said solution to obtain water dispersion. Lactose monohydrate, ferric oxide, and maize starch were granulated with said water dispersion in a fluid bed granulator. After the granulation step calcium carboxymethyl cellulose and magnesium stearate were added to the granules to obtain the granulated blend. Both blends were compressed into bilayer tablets.

### Example 11

Stability results of comparative example 1 were compared with stability results of example 5.

Samples produced as described in comparative example 1 were stored in closed glass vials which simulated conditions in Alu/Alu blister which was selected for the packaging of the final product. Significant increase of amlodipine degradation impurity (product of Maillard reaction with lactose) was observed already after 1 month on accelerated conditions 40°C/75%RH. It could be extrapolated that the increase of Maillard product in the formulation would exceed the qualification limit for impurities in the final product according to the ICH guidelines Q3B.

| Storage conditions | Imp D | Maillard | Amlodipine unknown | Amlodipine total | Imp-B | Imp F | Candesartan unknown | Candesartan Total |
|---|---|---|---|---|---|---|---|---|
| To | b.r.l. | b.r.l. | b.r.l. | b.r.l. | b.r.l | b.r.l | b.r.l | b.r.l. |
| 40°C/75%RH/ 1month/Closed vial | b.r.l. | 0,18 | b.r.l. | 0,18 | 0,11 | b.r.l | b.r.l | 0,11 |

| | | | | | | | | |
|---|---|---|---|---|---|---|---|---|
| Imp D: 3-ethyl 5-methyl 2-[(2-aminoethoxy)methyl]-4-(2-chlorophenyl)-6- methylpyridine-3,5-dicarboxylate Imp-B: 1-(cyclohexyloxycarbonyloxy)ethyl 3-((2'-(1H-tetrazol-5-yl)biphenyl-4-yl)methyl)-2-oxo-2,3-dihydro-1H-benzo[d]imidazole-4-carboxylate Imp F: 1-(cyclohexyloxycarbonyloxy)ethyl 2-ethoxy-1-((2'-(1-ethyl-1H-tetrazol-5-yl)biphenyl-4-yl)methyl)-1H-benzo[d]imidazole-7 -carboxylate b.r.l.: below reporting limit | | | | | | | | |

Samples produced as described in experiment 5 were stored in Alu/Alu blisters for 3 months on accelerated condition 40°C/75%RH. All of the amlodipine related compounds were below reporting treshold confirming better stability of example 5 tablets.

| Storage conditions | Imp D | Other indiv. | Amlodipine total | Imp-B | Imp F | Other indivi. | Candesartan Total |
|---|---|---|---|---|---|---|---|
| TO | b.r.l. | b.r.l. | b.r.l. | b.r.l | b.r.l | b.r.l | b.r.l. |
| 40°C/75%RH/ 3months/Alu-Alu blisters | b.r.l. | b.r.l. | b.r.l. | 0,22 | 0.19 | b.r.l | 0.41 |

| | | | | | | | |
|---|---|---|---|---|---|---|---|
| Imp D: 3-ethyl 5-methyl 2-[(2-aminoethoxy)methyl]-4-(2-chlorophenyl)-6- methylpyridine-3,5-dicarboxylate Imp-B: 1-(cyclohexyloxycarbonyloxy)ethyl 3-((2'-(1H-tetrazol-5-yl)biphenyl-4-yl)methyl)-2-oxo-2,3-dihydro-1H-benzo[d]imidazole-4-carboxylate Imp F: 1-(cyclohexyloxycarbonyloxy)ethyl 2-ethoxy-1-((2'-(1-ethyl-1H-tetrazol-5-yl)biphenyl-4-yl)methyl)-1H-benzo[d]imidazole-7 -carboxylate | | | | | | | |

### Examples 12 and 13 (comparative examples)

| Example | 12 | 13 |
|---|---|---|
| Candesartan Cilexetil | 32.0 mg | 32.0 mg |
| Lactose Monohydrate | 161.88 mg | 161.88 mg |
| Maize Starch | 40.0 mg | 40.0 mg |
| Ferric oxide red | 0.12 mg | 0.12 mg |
| Macrogol 8000 | 5.2 mg | / |
| Triehtyl citrate | / | 5.2 mg |
| Hydroxypropyl cellulose (Klucel^{™} EF) | 16.0 mg | 16.0 mg |
| Water, Purified* | q.s. | q.s. |
| Calcium carboxymethyl cellulose | 4.0 mg | 4.0 mg |
| Magnesium Stearate | 0.80 mg | 0.80 mg |
| Amlodipine besylate | 13.9 mg | 13.9 mg |
| Cellulose, Microcrystalline | 180.9 mg | 180.9 mg |
| Starch, Pregelatinised | 60.0 mg | 60.0 mg |
| Sodium Starch Glycolate | 21.0 mg | 21.0 mg |
| Silica, Colloidal Anhydrous | 1.3 mg | 1.3 mg |
| Magnesium Stearate | 3.0 mg | 3.0 mg |

Amlodipine besylate, microcrystalline cellulose, pregelatinised starch, sodium starch glycolate, colloidal anhydrous silica and magnesium stearate were homogeneously mixed to obtain the powder blend. Separately hydroxypropyl cellulose was dissolved in water, then Macrogol 8000 (Example 12) or triethyl citrate (Example 13) was added and candesartan cilexetil was dispersed in said solution to obtain water dispersion. Lactose monohydrate, ferric oxide, and maize starch were granulated with said water dispersion in a fluid bed or high shear granulator. After the granulation step calcium carboxymethyl cellulose and magnesium stearate were added to the granules to obtain the granulated blend. Both blends were compressed into bilayer tablets.

### Example 14 and 15 (comparative examples)

| Example | 14 | 15 |
|---|---|---|
| Candesartan Cilexetil | 16.0 mg | 16.0 mg |
| Lactose Monohydrate | 80.94 mg | 80.94 mg |
| Maize Starch | 20.0 mg | 20.0 mg |
| Ferric oxide red | 0.06 mg | 0.06 mg |
| Ferric oxide yellow | 0.03 mg | 0.03 mg |
| Macrogol 8000 | 2.6 mg | / |
| Triethyl citrate | / | 2.6 mg |
| Hydroxypropyl cellulose (Klucel^{™} EF) | 8.0 mg | 8.0 mg |
| Water, Purified* | q.s. | q.s. |
| Calcium carboxymethyl cellulose | 2.0 mg | 2.0 mg |
| Magnesium Stearate | 0.40 mg | 0.40 mg |
| Amlodipine besylate | 6.9 mg | 6.9 mg |
| Cellulose, Microcrystalline | 90.5 mg | 90.5 mg |
| Starch, Pregelatinised | 30.0 mg | 30.0 mg |
| Sodium Starch Glycolate | 10.5 mg | 10.5 mg |
| Silica, Colloidal Anhydrous | 0.6 mg | 0.6 mg |
| Magnesium Stearate | 1.5 mg | 1.5 mg |

Amlodipine besylate, microcrystalline cellulose, pregelatinised starch, sodium starch glycolate, colloidal anhydrous silica and magnesium stearate were homogeneously mixed to obtain the powder blend. Separately hydroxypropyl cellulose was dissolved in water, then Macrogol 8000 (Example 14) or triethyl citrate (Example 15) was added and candesartan cilexetil was dispersed in said solution to obtain water dispersion. Lactose monohydrate, ferric oxide, and maize starch were granulated with said water dispersion in a fluid bed or high shear granulator. After the granulation step calcium carboxymethyl cellulose and magnesium stearate were added to the granules to obtain the granulated blend. Both blends were compressed into bilayer tablets.

### Example 16 and 17 (comparative examples)

| Example | 16 | 17 |
|---|---|---|
| Candesartan Cilexetil | 16.0 mg | 16.0 mg |
| Lactose Monohydrate | 81.37 mg | 81.37 mg |
| Maize Starch | 20.0 mg | 20.0 mg |
| Ferric oxide red | 0.03 mg | 0.03 mg |
| Macrogol 8000 | 2.6 mg | / |
| Triethyl citrate | / | 2.6 mg |
| Hydroxypropyl cellulose (Klucel^{™} EF) | 4.0 mg | 4.0 mg |
| Water, Purified* | q.s. | q.s. |
| Calcium carboxymethyl cellulose | 5.6 mg | 5.6 mg |
| Magnesium Stearate | 0.40 mg | 0.40 mg |
| Amlodipine besylate | 13.9 mg | 13.9 mg |
| Cellulose, Microcrystalline | 59.1 mg | 59.1 mg |
| Starch, Pregelatinised | 18.0 mg | 18.0 mg |
| Sodium Starch Glycolate | 7.5 mg | 7.5 mg |
| Silica, Colloidal Anhydrous | 0.5 mg | 0.5 mg |
| Magnesium Stearate | 1.0 mg | 1.0 mg |

Amlodipine besylate, microcrystalline cellulose, pregelatinised starch, sodium starch glycolate, colloidal anhydrous silica and magnesium stearate were homogeneously mixed to obtain the powder blend. Separately hydroxypropyl cellulose was dissolved in water, then Macrogol 8000 (Example 16) or triethyl citrate (Example 17) was added and candesartan cilexetil was dispersed in said solution to obtain water dispersion. Lactose monohydrate, ferric oxide, and maize starch were granulated with said water dispersion in a fluid bed or high shear granulator. After the granulation step calcium carboxymethyl cellulose and magnesium stearate were added to the granules to obtain the granulated blend. Both blends were compressed into bilayer tablets.

## Claims

1. A pharmaceutical composition comprising candesartan cilexetil and amlodipine besylate having a composition selected from the embodiments a to f as specified in the table below,
| Embodiment | a [mg] | b [mg] | c [mg] | d [mg] | e [mg] | f [mg] |
|---|---|---|---|---|---|---|
| Candesartan Cilexetil | 16.0 | 16.0 | 16.0 | 16.0 | 16.0 | 16.0 |
| Lactose Monohydrate | 177.8 | / | 177.8 | 177.8 | 177.8 | 81.0 |
| Cellulose, Microcrystalline | / | 177.8 | / | / | / | / |
| Maize Starch | 40.0 | 40.0 | 40.0 | 40.0 | 40.0 | 20.0 |
| Ferric oxide yellow | 0.06 | 0.06 | 0.06 | 0.06 | 0.06 | 0.06 |
| Ferric oxide red | 0.12 | 0.12 | 0.12 | 0.12 | 0.12 | 0.12 |
| Macrogol 8000 | 5.2 | 5.2 | 5.2 | 5.2 | 5.2 | 2.6 |
| Hydroxypropyl cellulose (Klucel^{™} EF) | | | 16.0 | 16.0 | 16.0 | 8.0 |
| Hydroxypropyl cellulose (Klucel^{™} LF) | 16.0 | 16.0 | | | | |
| Water, Purified* | q.s. | q.s. | q.s. | q.s. | q.s. | q.s. |
| Calcium carboxymethyl cellulose | 4.0 | 4.0 | / | 4.0 | 4.0 | 2.0 |
| Sodium carboxymethyl cellulose | / | / | 4.0 | / | / | / |
| Magnesium stearate | 0.8 | 0.8 | 0.8 | 0.8 | 0.8 | 0.4 |
| Amlodipine besylate | 13.9 | 13.9 | 13.9 | 13.9 | 13.9 | 13.9 |
| Cellulose, Microcrystalline | 180.9 | 180.9 | 180.9 | 180.9 | 180.9 | 59.1 |
| Starch, Pregelatinised | / | 60.0 | 60.0 | 60.0 | 60.0 | 18.0 |
| Maize Starch | 60.0 | / | / | / | / | / |
| Sodium Starch Glycolate | 21.00 | 21.00 | 21.00 | 21.00 | / | 7.5 |
| Potassium polacrilin | / | / | / | / | 21.00 | / |
| Silica, Colloidal Anhydrous | 1.25 | 1.25 | 1.25 | 1.25 | 1.25 | 0.50 |
| Magnesium Stearate | 3.0 | 3.0 | 3.0 | 3.0 | 3.0 | 1.0 |
| | | | | | | |
|---|---|---|---|---|---|---|
| *Not present in the final dosage form. Evaporates during the process. | | | | | | |
which is obtainable by the following manufacturing method:
(i) homogeneously mixing amlodipine besylate, microcrystalline cellulose, binder (maize starch (embodiment a) or pregelatinised starch (embodiments b-f), sodium starch glycolate (embodiments a-d and f) or sodium carboxymethyl cellulose (embodiment e)), silica, colloidal anhydrous and magnesium stearate to obtain the powder blend;
(ii) separately dissolving hydroxypropyl cellulose in water, then adding Macrogol 8000 and dispersing candesartan cilexetil in said solution to obtain a water dispersion;
(iii) granulating lactose monohydrate (embodiments a and c-f) or microcrystalline cellulose (embodiment b), ferric oxide, and maize starch with said water dispersion in a fluid bed granulator;
(iv) adding calcium carboxymethyl cellulose (embodiments a-b and d-f) or sodium carboxymethyl cellulose (embodiment c) and magnesium stearate to the granules of step (iii) to obtain a granulated blend; and
(v) compressing both blends of steps (i) and (iv) into bilayer tablets.

2. A process for the preparation of a pharmaceutical composition according to claim 1 comprising:
(i) homogeneously mixing amlodipine besylate, microcrystalline cellulose, binder (maize starch (embodiment a) or pregelatinised starch (embodiments b-f), sodium starch glycolate (embodiments a-d and f) or sodium carboxymethyl cellulose (embodiment e)), silica, colloidal anhydrous and magnesium stearate to obtain the powder blend;
(ii) separately dissolving hydroxypropyl cellulose in water, then adding Macrogol 8000 and dispersing candesartan cilexetil in said solution to obtain a water dispersion;
(iii) granulating lactose monohydrate (embodiments a and c-f) or microcrystalline cellulose (embodiment b), ferric oxide, and maize starch with said water dispersion in a fluid bed granulator;
(iv) adding calcium carboxymethyl cellulose (embodiments a-b and d-f) or sodium carboxymethyl cellulose (embodiment c) and magnesium stearate to the granules of step (iii) to obtain a granulated blend; and
(v) compressing both blends of steps (i) and (iv) into bilayer tablets.

## Patentansprüche

1. Pharmazeutische Zusammensetzung, umfassend Candesartan-Cilexetil und Amlodipin-Besylat mit einer Zusammensetzung, die ausgewählt ist aus den Ausführungsformen a bis f, wie in der nachstehenden Tabelle angegeben,
| Ausführungsform | a [mg] | b [mg] | c [mg] | d [mg] | e [mg] | f [mg] |
|---|---|---|---|---|---|---|
| Candesartan Cilexetil | 16.0 | 16.0 | 16.0 | 16.0 | 16.0 | 16.0 |
| Lactose-Monohydrat | 177.8 | / | 177.8 | 177.8 | 177.8 | 81.0 |
| Cellulose, mikrokristallin | / | 177.8 | / | / | / | / |
| Maisstärke | 40.0 | 40.0 | 40.0 | 40.0 | 40.0 | 20.0 |
| Eisenoxid gelb | 0.06 | 0.06 | 0.06 | 0.06 | 0.06 | 0.06 |
| Eisenoxid rot | 0.12 | 0.12 | 0.12 | 0.12 | 0.12 | 0.12 |
| Macrogol 8000 | 5.2 | 5.2 | 5.2 | 5.2 | 5.2 | 2.6 |
| Hydroxypropylcellulose (Klucel^{™} EF) | | | 16.0 | 16.0 | 16.0 | 8.0 |
| Hydroxypropylcellulose (Klucel^{™} LF) | 16.0 | 16.0 | | | | |
| Wasser, gereinigt* | q.s. | q.s. | q.s. | q.s. | q.s. | q.s. |
| Calciumcarboxymethylcellulose | 4.0 | 4.0 | / | 4.0 | 4.0 | 2.0 |
| Natriumcarboxymethylcellulose | / | / | 4.0 | / | / | / |
| Magnesiumstearat | 0.8 | 0.8 | 0.8 | 0.8 | 0.8 | 0.4 |
| Amlodipinbesylat | 13.9 | 13.9 | 13.9 | 13.9 | 13.9 | 13.9 |
| Cellulose, mikrokristallin | 180.9 | 180.9 | 180.9 | 180.9 | 180.9 | 59.1 |
| Stärke, vorgelatiniert | / | 60.0 | 60.0 | 60.0 | 60.0 | 18.0 |
| Maisstärke | 60.0 | / | / | / | / | / |
| Natriumstärkeglykolat | 21.00 | 21.00 | 21.00 | 21.00 | / | 7.5 |
| Kaliumpolacrilin | / | / | / | / | 21.00 | / |
| Kieselsäure, kolloidal, wasserfrei | 1.25 | 1.25 | 1.25 | 1.25 | 1.25 | 0.50 |
| Magnesiumstearat | 3.0 | 3.0 | 3.0 | 3.0 | 3.0 | 1.0 |
| | | | | | | |
|---|---|---|---|---|---|---|
| *Nicht in der endgültigen Darreichungsform enthalten. Verdunstet während des Prozesses. | | | | | | |
die durch das folgende Herstellungsverfahren erhältlich ist:
(i) homogenes Mischen von Amlodipinbesylat, mikrokristalliner Cellulose, Bindemittel (Maisstärke (Ausführungsform a) oder vorgelatinierter Stärke (Ausführungsformen b-f), Natriumstärkeglykolat (Ausführungsformen a-d und f) oder Natriumcarboxymethylcellulose (Ausführungsform e)), kolloidaler wasserfreier Kieselsäure und Magnesiumstearat, um die Pulvermischung zu erhalten;
(ii) getrenntes Auflösen von Hydroxypropylcellulose in Wasser, anschließendes Hinzufügen von Macrogol 8000 und Dispergieren von Candesartan Cilexetil in dieser Lösung, um eine Wasserdispersion zu erhalten;
(iii) Granulieren von Lactose-Monohydrat (Ausführungsformen a und c-f) oder mikrokristalliner Cellulose (Ausführungsform b), Eisenoxid und Maisstärke mit der Wasserdispersion in einem Fließbettgranulierer;
(iv) Hinzufügen von Calciumcarboxymethylcellulose (Ausführungsformen a-b und d-f) oder Natriumcarboxymethylcellulose (Ausführungsform c) und Magnesiumstearat zu dem Granulat aus Schritt (iii), um eine granulierte Mischung zu erhalten; und
(v) Komprimieren beider Mischungen aus den Schritten (i) und (iv) zu Zweischichttabletten.

2. Verfahren zur Herstellung einer pharmazeutischen Zusammensetzung gemäß Anspruch 1, umfassend
(i) homogenes Mischen von Amlodipinbesylat, mikrokristalliner Cellulose, Bindemittel (Maisstärke (Ausführungsform a) oder vorgelatinierter Stärke (Ausführungsformen b-f), Natriumstärkeglykolat (Ausführungsformen a-d und f) oder Natriumcarboxymethylcellulose (Ausführungsform e)), kolloidaler wasserfreier Kieselsäure und Magnesiumstearat, um die Pulvermischung zu erhalten;
(ii) getrenntes Auflösen von Hydroxypropylcellulose in Wasser, anschließendes Hinzufügen von Macrogol 8000 und Dispergieren von Candesartan Cilexetil in dieser Lösung, um eine Wasserdispersion zu erhalten;
(iii) Granulieren von Lactose-Monohydrat (Ausführungsformen a und c-f) oder mikrokristalliner Cellulose (Ausführungsform b), Eisenoxid und Maisstärke mit der Wasserdispersion in einem Fließbettgranulierer;
(iv) Hinzufügen von Calciumcarboxymethylcellulose (Ausführungsformen a-b und d-f) oder Natriumcarboxymethylcellulose (Ausführungsform c) und Magnesiumstearat zu dem Granulat aus Schritt (iii), um eine granulierte Mischung zu erhalten; und
(v) Komprimieren beider Mischungen aus den Schritten (i) und (iv) zu Zweischichttabletten.

## Revendications

1. Composition pharmaceutique comprenant du candésartan cilexétil et du bésylate d'amlodipine présentant une composition sélectionnée parmi les modes de réalisation a à f spécifiés dans le tableau ci-dessous,
| Mode de réalisation | a [mg] | b [mg] | c [mg] | d [mg] | e [mg] | f [mg] |
|---|---|---|---|---|---|---|
| Candésartan cilexétil | 16,0 | 16,0 | 16,0 | 16,0 | 16,0 | 16,0 |
| Lactose monohydraté | 177,8 | / | 177,8 | 177,8 | 177,8 | 81,0 |
| Cellulose, microcristalline | / | 177,8 | / | / | / | / |
| Amidon de maïs | 40,0 | 40,0 | 40,0 | 40,0 | 40,0 | 20,0 |
| Oxyde de fer jaune | 0,06 | 0,06 | 0,06 | 0,06 | 0,06 | 0,06 |
| Oxyde de fer rouge | 0,12 | 0,12 | 0,12 | 0,12 | 0,12 | 0,12 |
| Macrogol 8000 | 5,2 | 5,2 | 5,2 | 5,2 | 5,2 | 2,6 |
| Hydroxypropylcellulos e (Klucel^{™} EF) | | | 16,0 | 16,0 | 16,0 | 8,0 |
| Hydroxypropylcellulos e (Klucel^{™} LF) | 16,0 | 16,0 | | | | |
| Eau, purifiée* | q.s. | q.s. | q.s. | q.s. | q.s. | q.s. |
| Carboxyméthylcellulos e de calcium | 4,0 | 4,0 | / | 4,0 | 4,0 | 2,0 |
| Carboxyméthylcellulos e de sodium | / | / | 4,0 | / | / | / |
| Stéarate de magnésium | 0,8 | 0,8 | 0,8 | 0,8 | 0,8 | 0,4 |
| Bésylate d'amlodipine | 13,9 | 13,9 | 13,9 | 13,9 | 13,9 | 13,9 |
| Cellulose, microcristalline | 180,9 | 180,9 | 180,9 | 180,9 | 180,9 | 59,1 |
| Amidon, prégélatinisé | / | 60,0 | 60,0 | 60,0 | 60,0 | 18,0 |
| Amidon de maïs | 60,0 | / | / | / | / | / |
| Glycolate d'amidon sodique | 21,00 | 21,00 | 21,00 | 21,00 | / | 7,5 |
| Polacriline potassium | / | / | / | / | 21,00 | / |
| Silice, colloïdale anhydre | 1,25 | 1,25 | 1,25 | 1,25 | 1,25 | 0,50 |
| Stéarate de magnésium | 3,0 | 3,0 | 3,0 | 3,0 | 3,0 | 1,0 |
| | | | | | | |
|---|---|---|---|---|---|---|
| *Ne figure pas dans la forme posologique finale. S'évapore au cours du processus. | | | | | | |
qui peut être obtenue par le procédé de fabrication suivant :
(i) le mélange homogène de bésylate d'amlodipine, de cellulose microcristalline, d'un liant (amidon de maïs (mode de réalisation a) ou amidon prégélatinisé (modes de réalisation b-f), glycolate d'amidon sodique (modes de réalisation a-d et f), ou carboxyméthylcellulose de sodium (mode de réalisation e)), de silice, colloïde anhydre et de stéarate de magnésium pour obtenir le mélange en poudre ;
(ii) la dissolution séparée de l'hydroxypropylcellulose dans de l'eau, puis l'ajout de Macrogol 8000 et la dispersion de candésartan cilexétil dans ladite solution pour obtenir une dispersion aqueuse ;
(iii) la granulation de lactose monohydraté (modes de réalisation a et c-f) ou de cellulose microcristalline (mode de réalisation b), d'oxyde de fer, et d'amidon de maïs avec ladite dispersion aqueuse dans un granulateur à lit fluidisé ;
(iv) l'ajout de carboxyméthylcellulose de calcium (modes de réalisation a-b et d-f) ou de carboxyméthylcellulose de sodium (mode de réalisation c) et de stéarate de magnésium aux granulés de l'étape (iii) pour obtenir un mélange granuleux ; et
(v) la compression des deux mélanges des étapes (i) et (iv) en comprimés bicouches.

2. Processus destiné à la préparation d'une composition pharmaceutique selon la revendication 1 comprenant :
(i) le mélange homogène de bésylate d'amlodipine, de cellulose microcristalline, d'un liant (amidon de maïs (mode de réalisation a) ou amidon prégélatinisé (modes de réalisation b-f), glycolate d'amidon sodique (modes de réalisation a-d et f), ou carboxyméthylcellulose de sodium (mode de réalisation e)), de silice, colloïde anhydre et de stéarate de magnésium pour obtenir le mélange en poudre ;
(ii) la dissolution séparée de l'hydroxypropylcellulose dans de l'eau, puis l'ajout de Macrogol 8000 et la dispersion de candésartan cilexétil dans ladite solution pour obtenir une dispersion aqueuse ;
(iii) la granulation de lactose monohydraté (modes de réalisation a et c-f) ou de cellulose microcristalline (mode de réalisation b), d'oxyde de fer, et d'amidon de maïs avec ladite dispersion aqueuse dans un granulateur à lit fluidisé ;
(iv) l'ajout de carboxyméthylcellulose de calcium (modes de réalisation a-b et d-f) ou de carboxyméthylcellulose de sodium (mode de réalisation c) et de stéarate de magnésium aux granulés de l'étape (iii) pour obtenir un mélange granuleux ; et
(v) la compression des deux mélanges des étapes (i) et (iv) en comprimés bicouches.
